# EUROPEAN PATENT SPECIFICATION

(11) **EP 3 018 135 B1**
(45) Date of publication and mention of the grant of the patent: **06.05.2020**
(21) Application number: 15192493.3
(22) Date of filing: 02.11.2015
(51) Int. Cl.: C07F 5/02, A61K 31/498, A61P 35/00, A61K 31/4745

(54) **A COMPOUND WITH ANTI-CARCINOGENIC EFFECT AND A METHOD FOR PRODUCTION OF THIS COMPOUND**
VERBINDUNG MIT ANTIKARZINOGENEM EFFEKT UND VERFAHREN ZUR HERSTELLUNG DIESER VERBINDUNG
COMPOSÉ À EFFET ANTI-CARCINOGÈNE ET PROCÉDÉ DE PRODUCTION DE CE COMPOSÉ

(30) Priority: 04.11.2014 TR 201412953
(43) Date of publication of application: 11.05.2016
(73) Proprietor: Ulusal Bor Arastirma Enstitusu, 06520 Ankara (TR)
(72) Inventor: OZTURK, Yusuf, 26470 ESK SEH R (TR); BENKLI, Kadriye, 26470 ESK SEH R (TR); DIKMEN, Miris, 26470 ESKISEH R (TR); CANTURK, Zerrin, 26470 ESKISEH R (TR)
(74) Representative: Dericioglu, E. Korhan

(56) References cited:
- US-A1- 2012 289 699
- CHETANA P R ET AL: "New ternary copper(II) complexes of l-alanine and heterocyclic bases: DNA binding and oxidative DNA cleavage activity", INORGANICA CHIMICA ACTA, ELSEVIER BV, NL, vol. 362, no. 13, 15 October 2009 (2009-10-15), pages 4692-4698, XP026642377, ISSN: 0020-1693, DOI: 10.1016/J.ICA.2009.06.024 [retrieved on 2009-06-23]

## Description

### Technical Field

This invention relates to a compound with anti-carcinogenic effect affecting cancer cell lines and inducing apoptosis in these cells by means of its boron content, and thus preventing the tumours in the patient's body from growing, and a method for production of this compound.

### Background of the Invention

There are many studies that indicate boron compounds induce apoptosis in uncontrollably growing cancer cells, causing death of these cells, thus inhibiting tumour growth, and acting as proteasome inhibitors.

Recent in-vitro studies report that Bortezomib inhibits NF-kB and induces apoptosis in multiple myeloma and prostate cells ( 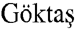 et al, 2010; Lashinger et al, 2005; An et al, 2003). It is reported that proteasome inhibitors (Bortezomib and second generation proteasome inhibitors (MLN9708/2238) inhibit NF-kB activity (Fuchs, 2013).

In a study conducted by Chauhan et al. on MM.1S cells with a proteasome inhibitor (MLN2238), NF-kB levels for MLN2238 in a concentration of 12 nmol/mL were determined by ELIZA method and significant decreases in NF-kB levels were identified at 12^{th} and 24^{th} hours of incubation (Chauhan et al, 2011).

In another study with Bortezomib NF-kB inhibition was observed in HCT116 and HT-29 colon cancer cells, while it was not observed in Caco2 cells, in result of the treatment (Voutsadakis et al, 2010). In a normal cell, c-myc is a transcription factor acting as a proto-oncogene. It is known that in some hematologic malignancy cases (Burkit Lymphoma) c-myc transforms into an oncogene and inclines the cell into continuous division. It is also known expression of c-myc oncogene is increased in cancerous cells. On the other hand, a decrease in c-myc expression inclines the cell to apoptosis (Thompson, 1998).

The lack of medicine more effective on chronic or acute leukaemia diseases, and the fact that current medicines are not yet effective on multiple myeloma condition, as well as the ongoing argument about their side effects, constitute some of the largest problems in the state of art. In addition, medicines used in treatment of leukaemia should selectively accumulate in leukaemia cells and only affect these cells. However, the compounds currently used or researched have not yet achieved a sufficient effect in this context.

Chetana et al. in Inorganica Chimica Acta 2009, 362(13), 4692-4698 disclose a 1,10-Phenanthrolin-5,6-dione-based copper complex having DNA binding activity. US2013/0289699 discloses Bortezomib as anti-neoplastic agent context.

### Summary of the Invention

The objective of the invention is to provide a compound affecting leukaemia and myeloma cell lines, thus preventing the tumour tissues in the patient's body from growing.

Another objective of the invention is to provide compound with an anti-carcinogenic effect, accumulating in diseased cells and selectively inducing cell apoptosis by means of its boronophenyl alanine (BPA) content.

Another objective of the invention is to provide a compound with a high activation rate on diseased cells by means of aromatic structures in its content. Another objective of the invention is to provide a compound exhibiting lipophilic effect by means of the phenyl ring and ethyl in its content, thus easily dispersing in the diseased cells.

Another objective of the invention is to provide a production method for a compound with the characteristics detailed above.

### Detailed Description of the Invention

The inventive, a compound with anti-carcinogenic effect, and a method of for its production, are illustrated in the accompanying figures, wherein;

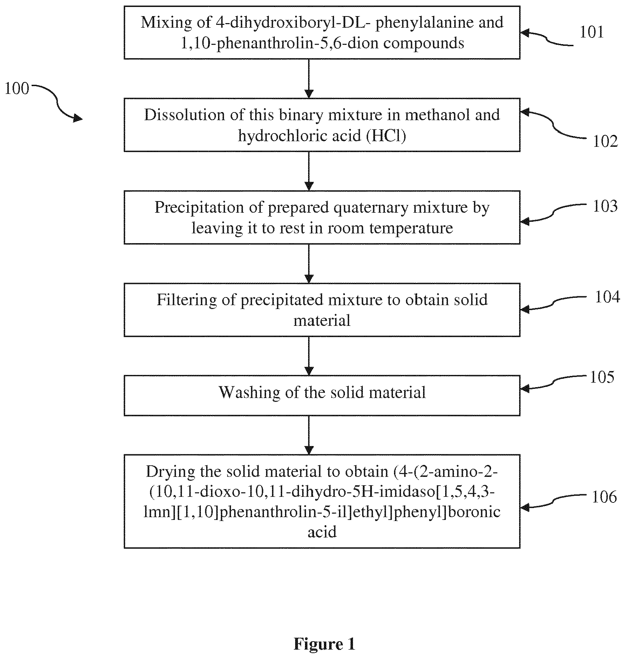

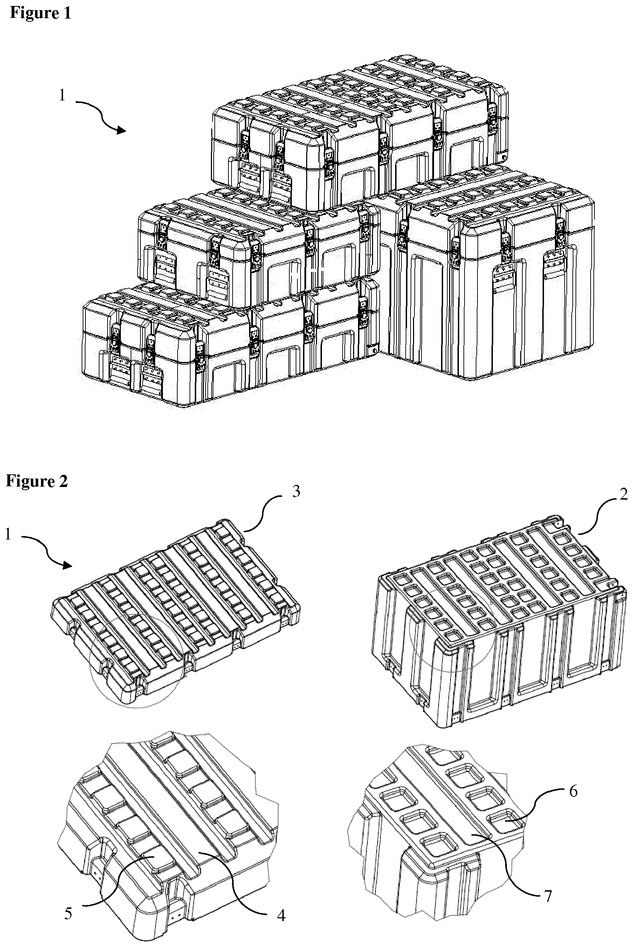

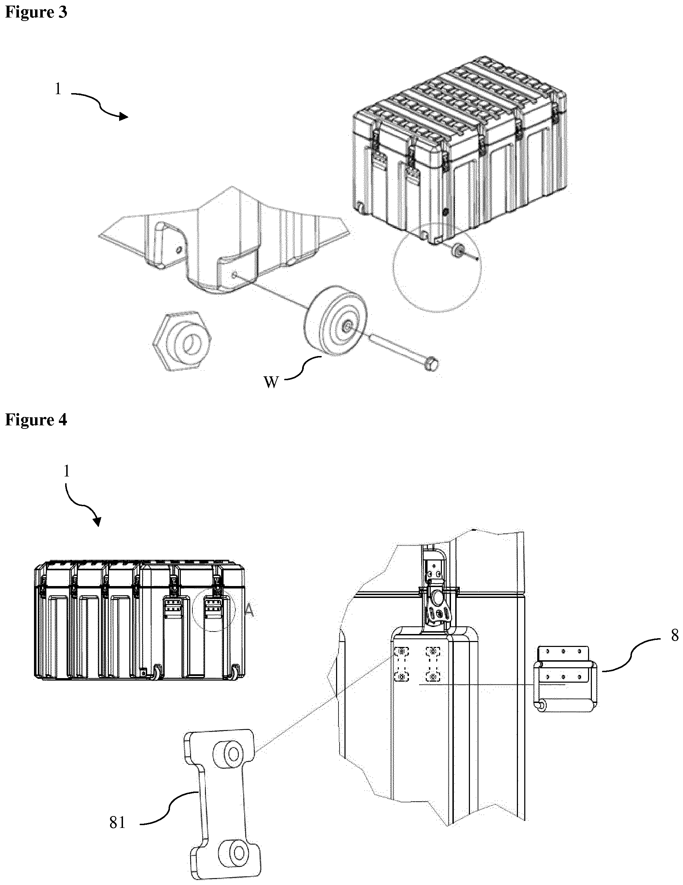

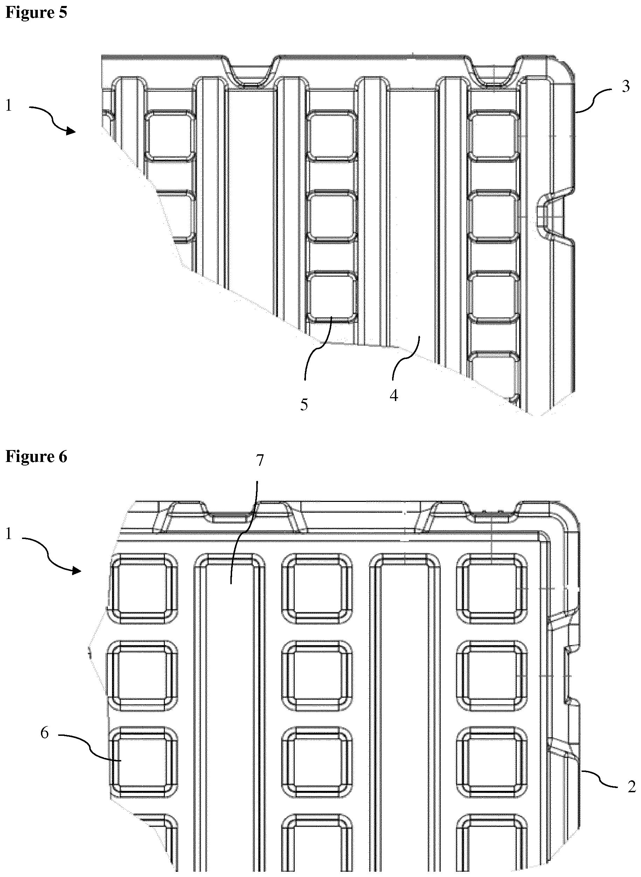

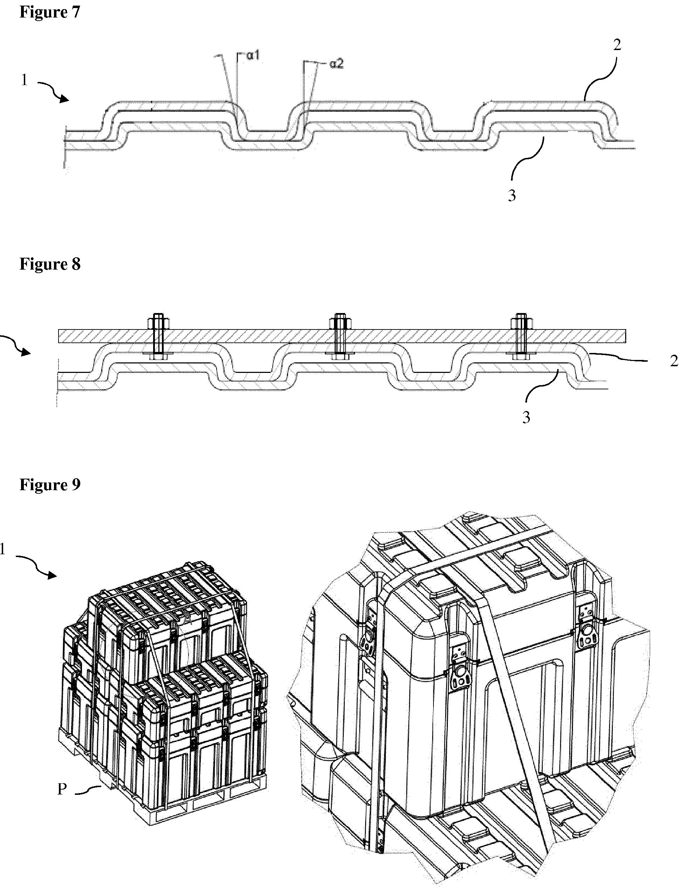

Figure 1 is a Flow chart for the inventive method.

A method (100) for production of a compound with anti-carcinogenic effect in order to achieve the inventive, essentially comprised of;
- Mixing of 4-dihydroxiboryl-DL-phenylalanine and 1,10-phenanthrolin-5,6-dion compounds (101),
- Dissolution of this binary mixture in methanol and hydrochloric acid (HC1) (102),
- Precipitation of prepared quaternary mixture by leaving it to rest in room temperature (103),
- Filtering of settled mixture to obtain solid material (104),
- Washing of the solid material (105),
- Drying the solid material to obtain (4-(2-amino-2-(10,11-dioxo-10,11-dihydro-5H-*imidazo*[1,*3*,4,*5*-lmn][1,10]*phenanthrolin*-5-*yl*)ethyl)phenyl)boronic acid (106).

In the inventive method (100) firstly the active ingredients 4-dihydroxiboryl-DL-phenylalanine (BPA) and 1,10-phenanthrolin-5,6-dion are mixed in a 1:1 molar ratio (101). 0.1 mole of 4-dihydroxiboryl-DL-phenylalanine (BPA) and 0.1 mole of 1,10-phenanthrolin-5,6-dion are used in the preferred embodiment of the invention. Then this binary mixture is added into 10 ml of methanol and 1 ml of 0,1 N HCl and stirred for 8 hours in a water bath at 30-40°C to dissolve (102). During this period of time reactions take place in the mixture and create the intended compound. After it becomes homogenous the quaternary mixture is left to precipitate in room temperature for 10 to 20 hours in order to have the solid material in the mixture settle (103)

The precipitated solid material is separated by means of dehydrated sodium filtering (104), and the separated solid material is washed with 10 ml of 5% NaHCO₃ (105). Finally, the compound obtained in result of washing process (105) is dried in a vacuum desiccator to obtain (4-(2-amino-2-(10,11-dioxo-10,11-dihydro-5H-*imidazo*[1,*3*,4,*5-*1mn*][*1,10]*phenanthrolin*-5-*yl)*ethyl*)*phenyl*)*boronic acid (106).

BPA molecule can bond at β section of 20S proteasome and inhibit this section by means of its atoms and groups with electro-negative character. This is associated with the oxo groups in the compound structure. In addition, the planar structure of the said molecule and the aromaticity arising from the resonance on the ring also increase activation of the compound obtained by the inventive method.

In addition, the phenyl ring and ethyl bridge in the said molecule contribute to lipophilic interaction of the inventive compound; non-bonded electron couples carried both by the nitrogen atoms on the ring and the nitrogen atom belonging to the amine bridge cause formation of dipole-dipole and ion-dipole bonds.

The hydroxyls in the dihydroxiboryl group allows formation of hydrogen bonds. BPA is generally interpreted as an amino acid analogue and passes the cell membrane with the L-amino acid carrier system. In addition, BPA intake is dependent on metabolic condition, and due to the higher metabolic activity rate of tumour cells compared to normal cells it is inclined to accumulate in tumour tissues.

The BPA molecule in the content of inventive compound can disintegrate again after entering the cell and it is easily accepted by the cells due to having an amino acid structure the separated boronophenyl alanine (BPA), joining tumour proteins and selectively accumulating in tumour cells.

In order to determine anti-carcinogenic effect of the inventive compound acute promyelocytic leukaemia (HL-60) and chronic myelogenous leukaemia (K-562) cells were used to prepare cancer cell cultures under suitable conditions. Both leukemic cell lines were subjected to the following methods:
- Determination of cytotoxicity / MTT test;
- Determination of apoptotic effect using flow cytometry device with Annexin V-FITC/7-AAD kit;
- Determination of apoptotic effect using flow cytometry device with caspase-3/7 activation;
- Determination of mitochondrial membrane integrity of cells using JC-1 dyeing method;
- Determination of apoptotic DNA using DNA fragmentation method with agarose gel;
- Determination of NF-kB and c-myc mRNA expression levels using real time PCR (polymerase chain reaction) method;
- Gene panel study using RT-PCR method.

The genes used in gene panel study with RT-PCR method include; NFKB-1, TNF, RELA, NMI, TP53, IL-6, TP53, PIK3CA, NFKB-2, BCL-3, NFKBIA, RELB, ICAM1, IL-8, KRAS, BCR, GAPDH.

In result of the conducted analyses it is found that the inventive compound exhibits significant cytotoxic effects on both HL-60 and K562 cells. IC₅₀ values of the compound on HL-60 and K562 cell were calculated as 2.16 and 2.62 µM at 24^{th} hour, and 2.85 and 3.41 µM at 48^{th} hour, respectively.

According to Annexin-V/7-AAD analysis with flow cytometry method, the inventive compound has not displayed early apoptotic effect on HL-60 cell line, but necrotic cell rates have exhibited an increase compared to control in concentrations of 3.12 µM and more. In case of K562 cells, it is found that early-late apoptotic and necrotic cell rates exhibit an increase compared to control in concentrations of 6.5 and 12.5 µM. Especially, in 12.5 µM concentration early and late apoptotic cell rate was analysed as 38.2%.

The inventive compound has caused an increase in the ratio of JC-1 positive cells among HL-60 cells starting from 1.5 µM (37.1% at 12.5 µM), while it caused a similar increase among K562 cells at 12.5 µM (14.7%). According to Annexin-V/7-AAD analysis results, even though the inventive compound exhibit apoptotic effects on K562 cells, the ratio of JC-1 positive cells among HL-60 cells was more significant in comparison to the control.

According to Annexin-V/7-AAD and caspase 3/7 analysis results the compound has caused a significant apoptotic effect on K562 cells in comparison to HL-60 cells. Especially, increase in the ratio of caspase 3/7 positive cells in comparison to the control was found to be larger in K562 cells.

At the end of 24 hours of incubation of the inventive compound (in a concentration of 12.5 µM) the increase in the ratio of caspase 3/7 positive cells was found to be 2 times more in K562 cells in comparison to HL-60 cells.

In a flow cytometry analysis study apoptotic effects on K562 cell caused by the inventive compound and Bortezomib used in current technique were compared. According to analysis results, the fact that the inventive compound has increased the apoptotic effect in K562 cells at the concentrations of 1.5, 3.12, 6.25 and 12.5 µM (at 24^{th} hour) are among the significant findings. While a concentration of 3.12 µM of Bortezomib, a proteasome inhibitor, exhibits an early apoptotic cell ratio of 18.1% in K562 cells at 24^{th} hour, the inventive compound in a concentration of 1.5 µM was found to cause an apoptotic cell ratio of 13.7%. Results of RT-PCR analysis have shown that concentrations of the inventive compound and Bortezomib decreases expression of NF-kB gene in K562 and HL-60 cells. On the other hand, c-myc expression in HL-60 cells was decreased, while c-myc expression was higher than control group values in K562 cells.

In result of gene panel analysis, it is found that in comparison to control and Bortezomib groups, the inventive compound decreases expression of NFkB-1, p53, PIK3CA, BCL-3, ICAM1, KRAS and BCR genes in HL-60 and K562 cells (downregulation). It was determined this decrease in gene expressions was more effective in HL-60 cells in comparison to K562 cells.

In conclusion of all these findings, it is determined that the inventive compound exhibits a stronger anti-carcinogenic effect in comparison to Bortezomib and similar compounds. The inventive anti-carcinogenic compound has decreased expression of some genes related to proteasome pathway (especially NFkB-1, p53, PIK3CA, BCL-3, ICAM1, KRAS and BCR) in both HL-60 and K562 cells, and caused anti-proliferative effects by means of various mechanisms. According to apoptosis results, it is seen that the inventive compound especially has a stronger apoptotic effect in K562 cells.

## Claims

1. A method for production (100) of a compound with anti-carcinogenic effect affecting cancer cell lines and selectively causing apoptosis in these cell, and thus preventing the tumours in the patient's body from growing, **characterised by;**
- Mixing of 4-dihydroxiboryl-DL-phenylalanine and 1,10-phenanthrolin-5,6-dion compounds (101),
- Dissolution of this binary mixture in methanol and hydrochloric acid (HC1) (102),
- Precipitation of prepared quaternary mixture by leaving it to rest in room temperature (103),
- Filtering of precipitated mixture to obtain solid material (104),
- Washing of the solid material (105),
- Drying the solid material to obtain *(4-(2-amino-2-(10,11-dioxo-10,11-dihydro-5H-imidazo[1,3,4,5-lmn][1,10]phenanthrolin-5-yl)ethyl)phenyl)boronic acid* (106).

2. A method for production (100) of a compound with anti-carcinogenic effect, as defined under Claim 1, **characterised by** mixing 4-dihydroxiboryl-DL-phenylalanine (BPA) and 1,10-phenanthrolin-5,6-dion compounds in a 1:1 molar ratio (101).

3. A method for production (100) of a compound with anti-carcinogenic effect, as under any one of the claims above, **characterised by** mixing of 0.1 mole of 4-dihydroxiboryl-DL-phenylalanine (BPA) and 0.1 mole of 1,10-phenanthrolin-5,6-dion (101).

4. A method for production (100) of a compound with anti-carcinogenic effect, as under any one of the claims above, **characterised by** addition of the mixture composed of BPA and 1,10-phenanthrolin-5,6-dion compounds into 10 ml of methanol and 1 ml of 0,1 N HCl and stirring for 8 hours in a water bath at 30-40°C to dissolve the mixture (102).

5. A method for production (100) of a compound with anti-carcinogenic effect, as under any one of the claims above, **characterised by** leaving the homogenous mixture composed by dissolving BPA and 1,10-phenanthrolin-5,6-dion mixture in methanol and HCl to precipitate in room temperature for 10 to 20 hours in order to have the solid material in the mixture settle (103).

6. A method for production (100) of a compound with anti-carcinogenic effect, as under any one of the claims above, **characterised by** filtration by using dehydrated sodium sulphate to obtain solid material (104).

7. A method for production (100) of a compound with anti-carcinogenic effect, as under any one of the claims above, **characterised by** washing of the separated solid material with 10 ml of 5% NaHCO₃ (105).

8. A method for production (100) of a compound with anti-carcinogenic effect, as under any one of the claims above, **characterised by** drying the compound obtained in result of washing process (105) in a vacuum desiccator to obtain *(4-(2-amino-2-(10,11-dioxo-10,11-dihydro-5H-imidazo[1,3,4,5-lmn][1,10]phenanthrolin-5-yl)ethyl)phenyl)boronic acid* (106).

9. A compound obtained by a method as under any one of the claims above, for use in cancer treatment due to its properties of selectively accumulating in cancer cells and inducing apoptosis in these cells.

## Patentansprüche

1. Verfahren zur Herstellung (100) einer Verbindung mit anti-karzinogener Wirkung, die Krebszelllinien beeinflusst und selektiv Apoptose in diesen Zellen bewirkt und dadurch das Wachstum von Tumoren im Körper des Patienten verhindert, **gekennzeichnet durch;**
- Mischen von 4-Dihydroxiboryl-DL-Phenylalanin und 1,10-Phenanthrolin-5,6-Dion-Verbindungen (101),
- Auflösung dieser binären Mischung in Methanol und Salzsäure (HCl) (102),
- Ausfällung einer zubereiteten quaternären Mischung, indem sie bei Raumtemperatur ruhen zulassen wird (103),
- Filtern der ausgefällten Mischung, um festes Material zu erhalten (104),
- Waschen des festen Materials (105),
- Trocknen des festen Materials, um *(4-(2-Amino-2-(10,11-dioxo-10,11-dihydro-5H-imidazo[1,3,4,5-lmn][1,10]phenanthrolin-5-yl)ethyl)phenyl)boronsäure* (106) zu erhalten.

2. Verfahren zur Herstellung (100) einer Verbindung mit anti-karzinogener Wirkung nach Anspruch 1, **gekennzeichnet durch** Mischen von 4-Dihydroxiboryl-DL-phenylalanin (BPA) und 1,10-Phenanthrolin-5,6-Dion-Verbindungen in einem Molverhältnis von 1:1 (101).

3. Verfahren zur Herstellung (100) einer Verbindung mit anti-karzinogener Wirkung, nach einem der vorstehenden Ansprüche, **gekennzeichnet durch** Mischen von 0.1 Mol 4-Dihydroxiboryl-DL-phenylalanin (BPA) und 0.1 Mol 1,10-Phenanthrolin-5,6-dion (101).

4. Verfahren zur Herstellung (100) einer Verbindung mit anti-karzinogener Wirkung, nach einem der vorstehenden Ansprüche, **gekennzeichnet durch** Zugabe der Mischung, die aus BPA und 1,10-Phenanthrolin-5,6-dion-Verbindungen besteht, in 10 ml Methanol und 1 ml 0,1 N HCl und Rühren für 8 Stunden in einem Wasserbad bei 30-40°C, um die Mischung (102) zu lösen.

5. Verfahren zur Herstellung (100) einer Verbindung mit anti-karzinogener Wirkung, nach einem der vorstehenden Ansprüche, **gekennzeichnet durch** Zulassen der homogenen Mischung, die durch Auflösen von BPA und einer 1,10-Phenanthrolin-5,6-Dion-Mischung in Methanol und HCl besteht, zur Ausfällung bei Raumtemperatur für 10 bis 20 Stunden, um das feste Material in der Mischung absetzen zu lassen (103).

6. Verfahren zur Herstellung (100) einer Verbindung mit anti-karzinogener Wirkung, nach einem der vorstehenden Ansprüche, **gekennzeichnet durch** Filtration unter Verwendung von dehydratisiertem Natriumsulfat, um festes Material (104) zu erhalten.

7. Verfahren zur Herstellung (100) einer Verbindung mit anti-karzinogener Wirkung, nach einem der vorstehenden Ansprüche, **gekennzeichnet durch** Waschen des abgetrennten festen Materials mit 10 ml 5% NaHCO₃ (105).

8. Verfahren zur Herstellung (100) einer Verbindung mit anti-karzinogener Wirkung, nach einem der vorstehenden Ansprüche, **gekennzeichnet durch** Trocknen der im Ergebnis des Waschprozesses (105) erhaltenen Verbindung in einem Vakuum-Exsikkator, um *(4-(2-Amino-2-(10,11-dioxo-10,11-dihydro-5H-imidazo[1,3,4,5-lmn][1,10]phenanthrolin-5-yl)ethyl)phenyl)boronsäure* (106) zu erhalten.

9. Verbindung, die durch ein Verfahren nach einem der vorstehenden Ansprüche erhalten wird, zur Verwendung in der Krebsbehandlung aufgrund ihrer Eigenschaften der selektiven Akkumulation in Krebszellen und der Induktion von Apoptose in diesen Zellen.

## Revendications

1. Méthode de production (100) pour un composé à effet anti-cancérigène affectant les lignées cellulaires cancéreuses et provoquant sélectivement l'apoptose dans ces cellules, et empêchant ainsi la croissance des tumeurs dans le corps du patient, **caractérisées par ;**
- Mélange de composés de 4-dihydroxyboryl-DL-phénylalanine et 1,10-phénanthroline-5,6-dion (101),
- Dissolution de ce mélange binaire dans méthanol et l'acide chlorhydrique (HC1) (102),
- Précipitation d'un mélange quaternaire préparé en le laissant reposer à température ambiante (103),
- Filtrage du mélange précipité pour obtenir de la matière solide (104),
- Lavage de la matière solide (105),
- Séchage de la matière solide pour obtenir *l'acide (4-(2-amino-2-(10,11-dioxo-10,11-dihydro-5H-imidazo[1,3,4,5-lmn][1,10]phénanthroline-5-yl)éthyl)phényl)boronique* (106).

2. Méthode de production (100) pour un composé à effet anti-cancérigène selon la revendication 1, **caractérisé par** mélange de composés de 4-dihydroxyboryl-DL-phénylalanine (BPA) et 1,10-phénanthroline-5,6-dion dans un rapport molaire de 1:1 (101).

3. Méthode de production (100) pour un composé à effet anti-cancérigène selon l'une quelconque des revendications précédentes, **caractérisé par** mélange de 0.1 mole de 4-dihydroxyboryl-DL-phénylalanine (BPA) et 0.1 mole de 1,10-phénanthroline-5,6-dion (101).

4. Méthode de production (100) pour un composé à effet anti-cancérigène selon l'une quelconque des revendications précédentes, **caractérisé par** ajout du mélange composé de composés de BPA et 1,10-phénanthroline-5,6-dion dans 10 ml de méthanol et 1 ml de 0,1 N HCl et agitation pendant 8 heures dans un bain-marie à 30-40°C pour dissoudre le mélange (102).

5. Méthode de production (100) pour un composé à effet anti-cancérigène selon l'une quelconque des revendications précédentes, **caractérisé par** laisser le mélange homogène composé en dissolvant le mélange de BPA et de 1,10-phénanthroline-5,6-dion dans méthanol et HCl pour précipiter à température ambiante pendant 10 à 20 heures afin de faire décanter la matière solide du mélange (103).

6. Méthode de production (100) pour un composé à effet anti-cancérigène selon l'une quelconque des revendications précédentes, **caractérisé par** la filtration en utilisant du sulfate de sodium déshydraté pour obtenir une matière solide (104).

7. Méthode de production (100) pour un composé à effet anti-cancérigène selon l'une quelconque des revendications précédentes, **caractérisé par** lavage de la matière solide séparée avec 10 ml de NaHCO₃ à 5% (105).

8. Méthode de production (100) pour un composé à effet anti-cancérigène selon l'une quelconque des revendications précédentes, **caractérisé par** séchage du composé obtenu à la suite du procédé de lavage (105) dans un dessiccateur sous vide pour obtenir *l'acide (4-(2-amino-2-(10,11-dioxo-10,11-dihydro-5H-imidazo[1,3,4,5-lmn][1,10]phénanthroline-5-yl)éthyl)phényl)boronique* (106).

9. Composé obtenu par une méthode selon l'une des revendications précédentes, pour utilisation dans le traitement du cancer en raison de ses propriétés d'accumulation sélective dans les cellules cancéreuses et d'induction de l'apoptose dans ces cellules.
